# EUROPÄISCHE PATENTSCHRIFT

(11) **EP 1 571 204 B1**
(45) Veröffentlichungstag und Bekanntmachung des Hinweises auf die Patenterteilung: **18.11.2009**
(21) Anmeldenummer: 04005177.3
(22) Anmeldetag: 04.03.2004
(51) Int. Cl.: C12N 5/00, C12N 5/08, C12N 5/06

(54) **Leukozytenstimulations-Matrix**
Leukocyte stimulation matrix
Matrice de stimulation de leucocytes

(43) Veröffentlichungstag der Anmeldung: 07.09.2005
(73) Patentinhaber: LeukoCare AG, 81379 München (DE)
(72) Erfinder: Scholz, Martin, Dr., 81379 München (DE)
(74) Vertreter: Strehl Schübel-Hopf & Partner

(56) Entgegenhaltungen:
- WO-A-96/27657
- WO-A-03/030965
- WO-A-2004/006951

## Beschreibung

### Gebiet der Erfindung

Die vorliegende Erfindung betrifft eine Leukozytenstimulations-Matrix, ein Leukozytenstimulations-Modul, welches die Leukozytenstimulations-Matrix umfasst, sowie ein Verfahren zur Leukozytenstimulation und/oder zur Induktion einer immunologischen Toleranz.

### Hintergrund der Erfindung

Die antigenspezifische Stimulation von Leukozyten ist ein wichtiges und wachsendes Forschungsgebiet zur Modulation von Immunreaktionen (Impfung, adoptive Immuntherapie u.s.w.). Derzeit sind in diesem Zusammenhang neben der Impfung im herkömmlichen Sinn vorwiegend therapeutische Ansätze zur ex vivo-Stimulation von Blutzellen bekannt.

Es existiert derzeit keine ausreichend funktionierende Therapie bei einer Vielzahl viraler chronischer Infektionserkrankungen. Die Chro-nizität basiert auf der Persistenz des viralen Antigens im Gewebe und der unzureichenden Immunantwort dagegen. Virale Erkrankungen werden zumeist mit Chemotherapeutika therapiert. Dies hat häufig eine Resistenzbildung der Viren und starke Nebenwirkungen zur Folge. Bisherige Experimente mit der ex vivo Stimulation von dendritischen Zellen und Effektorzellen mittels dendritischer Zellen (DC) z.B. bei Tumorerkrankungen waren nur bedingt erfolgreich (Cerundolo et al., Nature Immunology, Bd. 5, Nr. 1, S. 7-10, 2004). Vermutlich ist die ex vivo-Stimulation der Effektorzellen und die anschließende Rückfuhr in den Patienten zu störanfällig und zu schwach, um klinisch relevante Veränderungen zu induzieren. Darüber hinaus werden bei den derzeitig angewandten Methoden die dendritischen Zellen und Effektorzellen isoliert, die in sehr geringer Menge im Blut vorkommen. Die ex vivo Expansion dieser Zellen ist dabei ein weiterer störanfälliger Schritt.

Die antigenspezifische Stimulation der Leukozyten in Vollblut (in vivo) hat im Gegensatz zur ex vivo-Stimulation den Vorteil, dass alle physiologischen und essentiellen Faktoren des Bluts, die zur antigenspezifischen Leukozytenaktivierung notwendig sind, ausgenutzt werden können.

Die WO 00/27999 offenbart die Einbettung von hämatopoietischen Vorläuferzellen, Antigen-präsentierenden Zellen und lymphoretikulären Stromalzellen in eine poröse, feste Matrix, wobei die Matrix mit biologischen Mitteln beschichtet und mit einem gelartigen Mittel imprägniert sein kann. Diese Matrix kann zur Induzierung einer T-Zellreaktivität in vitro verwendet werden.

Die WO 93/20185 offenbart ein in vitro-Verfahren zur Proliferation von Vorläufern dendritischer Zellen.

WO 97/03186 (US 6,121,044) offenbart ein implantierbares Modul, welches eine Matrix mit eingebetteten dendritischen Zellen (DC) aufweist, mit dem eine primäre und sekundäre Immunantwort verursacht werden kann. Diese Veröffentlichung betrifft zwar auch ein in vivo verwendbares Modul, das Modul weist jedoch den Nachteil auf, dass eine Immunantwort mittels des dort offenbarten Moduls bzw. der Matrix nicht zeitlich gesteuert werden kann. Bei dieser Art der Stimulation ist es nicht gewährleistet, dass Leukozyten nach einer erfolgten Aktivierung kontrolliert von der stimulierenden Matrix abgelöst werden können, um zurück in den Blutstrom zu gelangen.

Banchereau und Steinmann beschreiben in Nature, Bd. 392, S.245-252, 1998 in einem Übersichtsartikel dendritische Zellen und deren Beteiligung an der Steuerung der Immunantwort.

Die WO 03/030965 offenbart ein Modul zur Leukozytenstimulation, wobei in dem Modul auf einem Träger ein Komplex aus Antigen-präsentierender Zelle (MHC) und Antigen immobilisiert ist. Dieses Dokument offenbart auch, dass eine Ablösung stimulierter Leukozyten erfolgt, es ist jedoch nicht offenbart, wie eine solche Ablösung kontrolliert gesteuert werden kann.

WO 03/031473 offenbart ein Modul zum Herabsetzen der Aktivität von Leukozyten, welches einen Träger und einen Liganden umfasst, der an den Träger gebunden ist und zur Wechselwirkung mit einem Rezeptor von Leukozyten geeignet ist.

WO 96/27657 offenbart ein Modul zur zeitlich kontrollierten Freisetzung von Substanzen aus einer bioabbaubaren Matrix.

Ziel der vorliegenden Erfindung ist es, eine Matrix oder eine die Matrix umfassende Vorrichtung bereitzustellen, mit der es möglich ist, in vivo Leukozyten zu stimulieren und/oder eine immunologische Toleranz im Blutkreislauf zu induzieren, wobei die stimulierten Leukozyten zeitlich kontrolliert wieder von der Matrix ablösbar sind.

### Gegenstand der Erfindung

Das oben erwähnte Problem der Erfindung wird durch eine Leukozytenstimulations-Matrix nach Anspruch 1 und ein Leukozytenstimulations-Modul gemäß Anspruch 12 gelöst.

Die Leukozytenstimulations-Matrix der vorliegenden Erfindung weist folgende Bestandteile auf:
a) ein oder mehrere Träger,
b) eine lösliche Matrix zur Einbettung eines oder mehrerer Bestandteile zur Generierung einer Leukozytenstimulation und/oder Induktion einer immunologischen Toleranz,
c) ein oder mehrere in die lösliche Matrix eingebettete Bestandteile zur Generierung einer Leukozytenstimulation und/oder Induktion einer immunologischen Toleranz.

Gemäß der vorliegenden Erfindung binden zirkulierende Antigen-spezifische Leukozyten an die in die lösliche Matrix eingebetteten Bestandteile, werden dort zeitweise gebunden, stimuliert und verlassen als aktive Zellen die Matrix bzw. ein Modul, welches die Leukozytenstimulations-Matrix enthält.

Durch eine derartige erfindungsgemäße Leukozytenstimulations-Matrix kann eine sukzessive kontrollierte Auflösung der äußeren Matrixoberfläche innerhalb eines vorbestimmten Zeitrahmens, bevorzugt innerhalb von Stunden bis zu etwa einem Tag, gewährleistet werden. Die lösliche Matrix löst sich kontrolliert schichtweise in einer Leukozyten enthaltenden Flüssigkeit, bevorzugt Vollblut, ab, wobei auch in der löslichen Matrix enthaltene Bestandteile zur Generierung einer Leukozytenstimulation und/oder Induktion einer immunologischen Toleranz (z.B. Antigene) abgelöst werden können, und hieran gebundene Leukozyten von der Matrix entfernt werden. Bei Einbringen der Leukozytenstimulations-Matrix bzw. eines Moduls, welches diese enthält, in den Körper werden diese Leukozyten durch die Blutzirkulation zurück in den Körper überführt. Die Auflösungszeit der jeweils äußeren Schichten der löslichen Matrix wird so gewählt, dass eine für die jeweilige antigenspezifische Leukozytenaktivierung geeignete Bindungsintensität und Bindungsdauer gewährleistet wird.

In einer bevorzugten Ausführungsform der vorliegenden Erfindung sind zusätzlich zu den Bestandteilen a) bis c) ein oder mehrere Bindungskoppler vorgesehen, um eine Bindung zwischen Träger und den ein oder mehreren Bestandteilen zur Generierung einer Leukozytenstimulation und/oder Induktion einer immunologischen Toleranz zu vermitteln. Bevorzugt handelt es sich bei der vermittelten Bindung um eine kovalente Bindung, so dass zumindest ein Teil der eingebetteten Bestandteile zur Generierung einer Leukozytenstimulation und/oder Induktion einer immunologischen Toleranz über die Bindungskoppler kovalent mit dem Träger verbunden ist. Erfindungsgemäß sind aber auch nicht-kovalente Bindungen, also z.B. ionische Bindungen, Bindungen aufgrund hydrophober Wechselwirkungen, van der Waals-Wechselwirkungen usw. dieses Bestandteils mit dem Bindungskoppler umfasst. Die vorliegende Erfindung umfasst auch solche Ausführungsformen, bei denen der Bestandteil zur Generierung einer Leukozytenstimulation und/oder Induktion einer immunologischen Toleranz sowohl an den Bindungskoppler gebunden ist, also auch teilweise ohne eine Bindung an den Bindungskoppler in die lösliche Matrix eingebettet ist.

### Träger

Der Träger der erfindungsgemäßen Leukozytenstimulations-Matrix ist nicht besonders eingeschränkt, solange auf diesem eine lösliche Matrix gemäß der vorliegenden Erfindung aufgebracht werden kann. Bevorzugt ist der Träger aus einem biokompatiblen Material. Bevorzugt sind Träger, die Poren aufweisen.

Als duroplastische Trägermaterialien sind bspw. Polyurethane, Polyamid oder Polyester verwendbar, wobei Polyurethane bevorzugt sind. Unter den Polyurethanen sind offenporige hydrophile, aber auch hydrophobe Polyurethan-Schäume verwendbar, die wahlweise Pigmente, z.B. Kohlenstoffpigmente und/oder Siliciumdioxidpigmente, enthalten können.

Weiterhin können als Polyurethane (PU) PU-Lacke verwendet werden oder andere Halbzeuge, die ebenfalls wahlweise Pigmente wie oben erwähnt enthalten können. Besonders bevorzugt ist PU Medical Grade (bezogen von der Firma KCI). Als thermoplastische Trägermaterialien sind z.B. Polycarbonate oder Polystyrol geeignet. Ebenfalls verwendbar sind Polyethylene oder Polypropylene, wobei diese bevorzugt in Kombination mit haftvermittelnden Pigmenten kombiniert werden. Auch Elastomere sind verwendbar.

Weitere geeignete Polymere sind z.B. PTFE (Polytetrafluorethylen), Dacron oder Polymethylpentan.

Weiterhin bevorzugt sind Materialien, die in der Chirurgie als sich auflösendes Nahtmaterial verwendet werden (Englisch: sutures), wie beispielsweise Monocryl (Poliglecaprone 25, PDS-2 (Polydioxanon), Maxon (Polyglyconat), Vicryl (Polyglactin-910) und Dexon-Plus (Polyglycolsäure). Die Verwendung derartiger, sich in Körperflüssigkeiten, beispielsweise Vollblut, auflösenden Materialien, bietet den Vorteil, dass sich nach vollständiger Auflösung der äußeren Matrix bzw. Hülle auch der Träger nach und nach auflöst, so dass bei Verwendung des Leukozytenstimulations-Moduls bzw. der Leukozytenstimulations-Matrix als Transplantat dieses nicht notwendigerweise aus dem Körper entfernt werden muss.

Weiterhin ist als Trägermaterial Glas in sämtlichen möglichen Formen, z.B. Fasern, offenporig oder geschäumt, geeignet.

Ebenfalls sind Metalle als Träger geeignet, bevorzugt biokompatible Metalle oder mit biokompatiblen Metallen beschichtete Träger. Auch Naturstoffe, wie Därme, oder biologische Materialien, wie Schwämme, sind erfindungsgemäß bevorzugt verwendbar.

Polymermaterialien, die Poren aufweisen, sind besonders bevorzugt, insbesondere Polyurethan. Die Poren könne eine beliebige Größe aufweisen. Durchschnittliche Porengrößen im Bereich von 0,5 - 2 mm, insbesondere etwa 1 mm, sind bevorzugt.

### Lösliche Matrix

Weiterhin umfasst die Leukozytenstimulations-Matrix der vorliegenden Erfindung eine auf dem Träger befindliche lösliche Matrix, in die ein oder mehrere in die Matrix eingebettete Bestandteile zur Generierung einer Leukozytenstimulation und/oder Induktion einer immunologischen Toleranz eingebettet sind. Der Begriff "löslich" bedeutet, so wie er in der vorliegenden Erfindung zur Beschreibung der löslichen Matrix verwendet wird, dass sich die lösliche Matrix in Vollblut innerhalb von Stunden bis zu wenigen Tagen auflöst.

Die lösliche Matrix (b) ist in einer bevorzugten Ausführungsform der vorliegenden Erfindung aus langkettigen Zuckerverbindungen wie z.B. Stärke, Cellulose und/oder Glykogen einerseits, und Polyethylenglykol andererseits aufgebaut. Erfindungsgemäß braucht aber keine langkettige Zuckerverbindung enthalten zu sein. PEG ist somit in einer bevorzugten Ausführungsform der Erfindung notwendiger Bestandteil der löslichen Matrix, und die langkettigen Zucker sind wahlweiser Bestandteil. In einer anderen bevorzugten Ausführungsform der vorliegenden Erfindung sind neben PEG keine langkettigen Zucker als Bestandteile der löslichen Matrix enthalten.

Die lösliche Matrix umfasst in der genannten Ausführungsform mit langkettigen Zuckerverbindungen bevorzugt 50-90 Gew.-%, bevorzugt 60-80 Gew.-%, langkettige Zuckerverbindung(en) und 10-50 Gew.-%, bevorzugt 20-40 Gew.-%, eines Polyethylenglykols, bezogen auf die Summe aus langkettiger Zuckerverbindung und Polyethylenglykol. Bei beiden genannten erfindungsgemäßen Ausführungsformen der löslichen Matrix kann gewährleistet werden, dass sich die lösliche Matrix innerhalb von wenigen Stunden, bevorzugt etwa 4-12 Stunden, im Blut langsam auflöst. Grundsätzlich kann durch Steigerung des PEG-Anteils die Auflösung verlangsamt werden. Somit ist eine Steuerung der Auflösungszeit der löslichen Matrix durch Variation der Anteile an PEG und langkettiger Zuckerverbindung möglich.

Eine Steuerung der Auflösungszeit der löslichen Matrix kann weiterhin auch über das Molekulargewicht des PEG gesteuert werden. Als Polyethylenglykol (PEG) wird bevorzugt PEG mit einem Molekulargewicht im Bereich von 1-200 kD verwendet. Bevorzugt ist ein Molekulargewichtsbereich von etwa 10 bis etwa 60 kD, weiterhin bevorzugt etwa 10-30kD, besonders bevorzugt etwa 30 kD. Es können auch modifiezierte PEG verwendet werden, bspw. solche, bei denen PEG-Moleküle durch Spacer verbunden sind. PEG wird bevorzugt als wässerige Lösung verwendet, wobei z.B. bei einem PEG von 15-20 kD Molekulargewicht etwa eine 1-10 Gew.-% Lösung verwendet wird, bevorzugt etwa 5 Gew.-%. Bei PEG mit kurzem Molekulargewicht (z.B. etwa 6 kD) kann die Konzentration bis zu 20 Gew.-% betragen, bei PEG mit größerem Molekulargewicht kann die Konzentration auch darunter liegen. Die geeignete Konzentration kann vom Fachmann durch Versuche ermittelt werden. PEG kann auch an verschiedene Zytokine, wie bspw. Interferone (IFN), gekoppelt werden, wobei derartige PEG-Interferonprodukte ebenfalls im Rahmen der vorliegenden Erfindung als Bestandteil der löslichen Matrix verwendet werden können. Damit hat man die Möglichkeit, Zytokine als Leukozyten-stimulierende Mittel mit in die lösliche Matrix einzubeziehen.

### Bestandteil zur Generierung einer Leukozytenstimulation und/oder Induktion einer immunologischen Toleranz

Leukozytenstimulation bedeutet erfindungsgemäß, dass bereits geprägte Immunzellen spezifisch in ihrer Immunantwort verstärkt werden. Der Begriff umfasst aber auch die Prägung ungeprägter (naiver) Immunzellen. Der Begriff Leukozyten umfasst B-Lymphozyten, T-Lymphozyten, Granuozyten und Neutrophile.

Unter Induktion einer Toleranz ist erfindungsgemäß zu verstehen, dass eine Anergie der Leukozyten gegenüber einem bestimmten Antigen induziert wird, d.h. eine Inaktivierung. Dabei werden Leukozyten mit einem Antigen stimuliert und gleichzeitig kostimulatorische Moleküle inhibiert.

Als Bestandteil zur Generierung bzw. Auslösung bzw. Erzeugung einer Leukozytenstimulation und/oder Induktion einer immunologischen Toleranz können erfindungsgemäß z.B. Antigene, Haptene, MHC-Moleküle, kostimulatorische Faktoren, Zellbestandteile und/oder Membranfragmente Antigen-präsentierender Zellen verwendet werden. Die Antigene brauchen dabei nicht vorher isoliert werden. Auch unversehrte oder weitgehend unversehrte Viren, Bakterien, Zellen oder Hüllen hiervon, welche Antigene aufweisen, können als Bestandteil zur Generierung einer Leukozytenstimulation und/oder Induktion einer immunologischen Toleranz erfindungsgemäß verwendet werden. Es können auch inaktivierte Viren, Bakterien usw. verwendet werden. Die Inaktivierung kann gemäß dem Fachmann bekannten Verfahren erfolgen, bspw. UV-Bestrahlung.Die Antigene, z.B. Peptide, die aus beispielsweise Viren-, Bakterien- oder Tumorzellpräparationen isoliert werden, können an MHC-Moleküle gekoppelt sein oder an die Membranbestandteile Antigen-präsentierender Zellen, wie beispielsweise dendritischer Zellen (DC), der zu behandelnden Patienten (autologe Zellen) oder können von allogenen Spendern stammen. Der immunrelevante Anteil der löslichen Matrix, d.h. der Teil, der eine Immunantwort hervorruft, kann erfindungsgemäß auch als immunstimulatorischer Komplex (IK) bezeichnet werden.

Bei dem Antigen kann es sich um ein synthetisches Antigen handeln, oder das Antigen wird aus Viren, Bakterien, Pilzen, Protozoen, Parasiten (z.B. Würmern), Tumoren, Allergenen, Zellkulturen oder aus körpereigenem Gewebe gewonnen. Das MHC-Molekül und/oder der kostimulatorische Faktor können aus körpereigenem Gewebe gewonnen werden, aus Zellkulturen, oder diese können synthetisch hergestellt werden.

Eine Übersicht der kostimulatorischen Moleküle findet sich z.B. in Rothstein und Sayegh, Immunolo-gical Reviews 2003, "T-cell costimulatory pathways in allo-graft reaction and tolerance". Diese sind erfindungsgemäß umfasst.

Erfindungsgemäß können in Anwesenheit von Antigen bevorzugt die folgenden kostimulatorischen Molemüle verwendet werden:
- Alle kostimulatorische Moleküle der CD28/CTLA-4:B7-Familie: CD28, CTLA-4, ICOS, PD-1, B7-1, B7-2, B7RP-1, PD-L1, PD-L2.
- Tumornekrosefaktoren:Tumornekrosefaktorrezeptorfamilie: CD154/CD40L, 4-IBB (CD137), Ox-40 (CD134), CD27; Liganden: CD40, 4IBBL (CD137L), Ox40L (CD134L), CD70.

Weitere kostimulatorische Moleküle, die noch gefunden werden, sind ebenfalls umfasst.

Bevorzugt ist der Bestandteil zur Generierung einer Leukozytenstimulation und/oder Induktion einer immunologischen Toleranz, z.B. gegenüber einem Virus, ein Virus der Familie der Herpesviren, insbesondere das Cytomegalievirus (CMV), Epstein Barr Virus (EBV) oder Herpes Simplex Virus (HSV 1+2), oder ein Fragment oder Teil, eine Virushülle oder ein Virushüllenfragment hiervon, die Antigene enthalten, welche eine Immunantwort hervorrufen können.

Weiterhin sind verwendbar: SARS (Coronavirus); Rhinovirus; Picornaviren, insbesondere Poliovirus, Coxsackievirus, Retroviren, insbesondere das humane Immundefizienz Virus (HIV),
ein Hepatitis auslösendes Virus, insbesondere Hepatitis B Virus (HBV) oder Hepatitis C Virus (HCV),
Coronaviren, insbesondere SARS-assoziierte Coronaviren; und/oder jeweils Antigene hiervon oder ein Fragment oder Teil, eine Virushülle oder ein Virushüllenfragment hiervon, die Antigene enthalten, welche eine Immunantwort hervorrufen können.

Weiterhin sind erfindungsgemäß alle anderen Viren, die mit chronischen Entzündungskrankheiten oder Tumorerkrankungen verbunden sind, Orthomyxoviren (Influenza), Paramyxoviren (Mumps, Masern bei fehlender oder nicht ausreichender Impfung), Papovaviren (Papillomaviren) verwendbar. Auch Viren, gegen die keine Impfung existiert und/oder mit akuter Pathogenität/Lethalität sind einsetzbar.

Besonders geeignet ist die erfindungsgemäße Leukozytenstimulations-Matrix für bei Patienten verwendbar, bei denen eine antivirale Chemotherapie nicht greift (z.B. Resistenzen entwickelt wurden) oder lokale Virus-vermittelte Entzündungen durch systemische Behandlung nicht erreicht werden (z.B. CMV-Retinitis).

Besonders bevorzugt ist das Antigen ein Cytomegalievirus des Stamms CMV Hi91, AD169, Towne, Davis oder die Hülle, ein Teil oder Fragment hiervon. Andere Labor- oder Wildstämme sind aber ebenfalls umfasst.

Als Virentypen können RNA-Viren, DNA-Viren, Viren mit Hüllen, Viren ohne Hüllen, onkogene Viren (Papilloma-Virus), HHV-8 u.s.w. verwendet werden.

Im Fall einer Antibiotika-Unverträglichkeit, Resistenzen oder lokalen Bakterien-induzierten lokalen Entzündungen können erfindungsgemäß Bakterien oder Teile bzw. Fragmente hiervon, die Antigene enthalten, verwendet werden.

Grundsätzlich sind alle Spezies aus den Familien bzw. Gattungen der Staphylokokken; Streptokokken; Enterokokken; Neisserien; Enterobakerien; Vibrionen (Cholera); alle nichtfermentierenden Bakterien; Campylobacter; Helicobacter; Haemophilus; Bordetellen; Legionellen; alle Anthropozoonoseerreger; Korynebakterien; Bacillus; Clostridien; Mykobakterien; Nocardien; Treponemen; Borrelien (bevorzugte zeitlich nahe Testung bei Patienten mit Borreliose); Leptospiren; Bartonella; Mykoplasmen; Chlamydien verwendbar.

Unter den Pilzen sind insbesondere Sproßpilze (Candida); Fadenpilzer (Schimmelpilze, Aspergillus); Dimorphe Pilze; und andere wie Pneumocystis carinii verwendbar, bevorzugt humanpathogene Pilze und sogenannte "Krankenhauskeime" wie Aspergillus oder Candida albicans.

Unter den Parasiten sind insbesondere Protozoen; Trematoden; Cestoden; Nematoden erfindungsgemäß einsetzbar.

Auch Prionen oder noch nicht bekannte oder definierte Pathogene sind grundsätzlich einsetzbar.

Im Fall von Tumoren können insbesondere Membranbestandteile inaktivierter Tumorzellen, besonders des malignen Melanoms, verwendet werden.

Bei Autoimmunerkankungen/Allergien können grundsätzlich erfindungsgemäß alle derzeit und zukünftig bekannten relevanten Antigene, besonders Kollagen, Zellmembranen von biliären Epithelzellen etc. verwendet werden.

Ein bevorzugter inhibitorischer Faktor, der bei der Erzeugung einer Immuntoleranz verwendet werden kann, ist LIR-1.

Die Konzentration des Bestandteils zur Generierung einer Leukozytenstimulation und/oder Induktion einer immunologischen Toleranz in der Hülle kann vom Fachmann durch geeignete Versuche ermittelt werden. Bevorzugt richtet sich die Menge bzw. Konzentration dieses Bestandteils bei Einbringen in den Körper nach den in herkömmlichen Impfungen verwendeten Mengen. Bei herkömmlichen Impfungen werden beispielsweise etwa 20 µg Virusantigen verwendet.

Insgesamt ist die lösliche Matrix zusammen mit dem eingebetteten Antigen erfindungsgemäß bevorzugt in etwa wie folgt aufgebaut:

### Definition der Matrix (Gew.-%):

| | Möglicher Bereich | Bevorzugter Bereich |
|---|---|---|
| Summe aus PEG und langkettigem Zucker | 99,5-99,999 Gew.-% | 99,9-99,99 Gew.-% |
| Antigen | 0,001-0,5 Gew.-% | 0,01-0,1 Gew.-% |

Wird statt eines im wesentlichen isolierten Antigens ein ganzes Virus, Bakterium, eine Hülle hiervon oder dergleichen verwendet, kann der oben angegebene Antigenanteil auch mehr als 0,5 Gew.-% oder 0,1 Gew.-% betragen

Wie oben bereits erwähnt, liegt ein langkettiger Zucker dabei nicht notwendigerweise vor. Die Verteilung aus PEG und langkettiger Zuckerverbindung beträgt in einer bevorzugten Ausführungsform jedoch 50-90 Gew.-%, bevorzugt 60-80 Gew.-%, langkettige Zuckerverbindung(en) und 10-50 Gew.-%, bevorzugt 20-40 Gew.-%, eines Polyethylenglykols, bezogen auf die Summe aus langkettiger Zuckerverbindung und Polyethylenglykol.

### Bindungskoppler

In einer bevorzugten Ausführungsform der vorliegenden Erfindung ist ein Bindungskoppler vorgesehen, um eine Bindung zwischen Träger und dem einen oder mehreren Bestandteilen zur Generierung einer Leukozytenstimulation und/oder Induktion einer immunologischen Toleranz zu vermitteln. Bei diesem Bindungskoppler, der bevorzugt eine kovalente Bindung vermittelt, handelt es sich vorzugsweise um ein Element, das aus Cyanogenbromid, Cyanoborhydrid, Agarose, Agarosederivaten, Silan, Silanderivaten oder Kombinationen hiervon ausgewählt ist. In einer weniger bevorzugten Ausführungsform kann auch p-Toluolsulfonylchlorid verwendet werden. Besonders bevorzugt ist das Silanderivat ein Alkoxysilan, noch bevorzugter ein Anhydridoalkoxysilan oder ein anderes Alkoxysilan, welches mindestens eine Carboxylgruppe aufweist. Die Alkoxygruppe ist jeweils bevorzugt eine Methoxy- oder Ethoxygruppe. Ein besonders bevorzugtes Anhydridoalkoxysilan ist 3-(Triethoxysilyl)propylbernsteinsäureanhydrid (GENIOSIL ® GF 20, Wacker). Auch Aminogruppen enthaltende Alkoxysilane sind verwendbar, z.B. (3-Aminopropyl)trimethoxysilan oder [3-(2-Aminoethylamino)propyl]trimethoxysilan. Weiterhin bevorzugt ist (3-(2,3-Epoxypropoxy)propyl)trimethoxysilan (GENIOSIL® GF 80). Weiter Alkoxysilane können entsprechend der jeweiligen Träger und Bedingungen vom Fachmann ausgewählt werden.

Es hat sich herausgestellt, dass Alkoxysilane, insbesondere Anhydridoalkoxysilane wie GENIOSIL® GF 20, aber auch GENIOSIL® GF 80, besonders gut zur Vermittlung einer Bindung an einen Polyurethan- oder Glasträger geeignet sind.

Die Erfindung erlaubt die zeitlich limitierte Bindung von Leukozyten aus Vollblut oder aus Vollblut isolierten Leukozyten, deren spezifische Antigenstimulation und die Rückführung der stimulierten Leukozyten in den Blutkreislauf durch Auflösung der Matrix im Vollblut bzw. einer Leukozyten enthaltenden physiologischen Flüssigkeit.

Weiterhin ist erfindungsgemäß die Induktion einer immunologischen Toleranz durch die Zugabe inhibitorischer Faktoren bei gleichzeitiger spezifischer Aktivierung möglich. Die Zugabe inhibitorischer Faktoren kann beispielsweise während der Bildung der löslichen Matrix erfolgen, d.h. die inhibitorischen Faktoren werden mit in die lösliche Matrix eingebettet, oder es kann eine Zugabe während der Leukozytenstimulation erfolgen. Die inhibitorischen Faktoren können auch über einen der oben genannten Bindungskoppler in die lösliche Matrix eingebettet werden. Im Fall einer Induktion einer immunologischen Toleranz wird ein Leukozyt zwar mit dem Antigen in Kontakt gebracht, kann aber durch die Einwirkung der inhibitorischen Faktoren gegen die Antigene nicht aktiv werden. Ein geeigneter inhibitorischer Faktor ist beispielsweise LIR-1. Weitere inhibitorische Faktoren sind dem Fachmann bekannt und durch die Erfindung umfasst. Auch inhibitorische Faktoren, die noch gefunden werden, sind erfindungsgemäß umfasst.

Die erfindungsgemäße Leukozytenstimulations-Matrix kann auch als Implantat verwendet werden.

### Leukozytenstimulationsmodul

Die Leukozytenstimulations-Matrix gemäß der vorliegenden Erfindung kann in einem Leukozytenstimulations-Modul eingesetzt werden. Das Leukozytenstimulations-Modul umfasst ein Gehäuse, welches bevorzugt aus Glas oder einem Kunststoff hergestellt ist, wobei der Kunststoff bevorzugt untoxisch und gegenüber einer biologischen Flüssigkeit wie Vollblut inert, d.h. im wesentlichen unlöslich, ist. Das Leukozytenstimulations-Modul umfasst mindestens eine Öffnung. Bevorzugt ist mindestens eine Einlassöffnung und mindestens eine Auslassöffnung vorgesehen, weiterhin bevorzugt genau eine Einlassöffnung und genau eine Auslassöffnung. Das Gehäuse bzw. Modul kann aber auch auf andere Weise ausgestaltet sein, beispielsweise nur mit einer Öffnung, durch welche eine Leukozyten enthaltende Flüssigkeit einströmt und auch wieder ausströmt. Weitere Ausgestaltungen des Moduls können vom Fachmann konstruiert werden, bspw. solche mit mehr als einer, z.B. zwei Eingangsöffnungen und/oder Ausgangsöffnungen.

Weiterhin betrifft die vorliegende Erfindung ein Verfahren zur Leukozytenstimulation und/oder Induktion einer immunologischen Toleranz, wobei eine Leukozyten enthaltende Flüssigkeit, wie beispielsweise Vollblut, ex vivo mit einer Leukozytenstimulations-Matrix der vorliegenden Erfindung in Kontakt gebracht wird. Bevorzugt erfolgt dieses Kontaktieren in einem Leukozytenstimulations-Modul gemäß der vorliegenden Erfindung.

Das erfindungsgemäße Modul kann bei Patienten bzw. in klinischen Situationen mit mangelnder zellulärer Immunantwort z. B. gegen virale oder bakterielle Infektionserreger oder Tumorantigene eingesetzt werden.

Das erfindungsgemäße Modul kann beispielsweise in den Blutstrom des Patienten eingesetzt werden. Eine bevorzugte Ausführungsform besteht darin, dass das Modul zum transienten Einbringen in den Patienten-Blutstrom über einen Sheldon-Katheter eingesetzt wird. Dabei binden zirkulierende Antigen-spezifische T-Zellen an die in die Matrix eingebetteten Bestandteile, werden dort zeitweise gebunden, stimuliert und verlassen als hochaktive Zellen das Modul. Effektorzellen mit nur unzureichender spezifischer Funktion werden stimuliert und verlassen als hoch aktive Zellen das Modul. Gedächtniszellen, die zuvor Kontakt mit dem in die lösliche Matrix eingebetteten Bestandteil hatten, binden ebenfalls an die Matrix. Auch diese bereits geprägten Zellen werden stark aktiviert. Weiterhin können ungeprägte (naive) T-Zellen an die Matrix binden und werden gegen das präsentierte Antigen MHC-Molekül, kostimulatorischen Faktor oder anderen Zellbestandteil geprägt. Durch kostimulatorische Faktoren, die dem Modul wahlweise beigegeben werden können, oder die ebenfalls in die lösliche Matrix eingebettet werden (z.B. DC-Adhäsionsmoleküle, Zytokine etc.), kommt es zur Aktivierung dieser Zellen, die zurück in den Blutkreislauf gelangen, um dort mittels spezifischer Effektormechanismen das Pathogen zu eliminieren. Die Induktion einer humoralen Immunantwort kann sich anschließen (T-Zell-vermittelte B-Zellaktivierung).

Eine Immuntoleranz kann wie oben beschrieben durch Anwendung einer entsprechenden Matrix , in die inhibierende Faktoren eingebettet sind, in dem Modul erreicht werden, oder indem inhibierende Faktoren während der Leukozytenstimulation in das Modul gegeben werden.

Durch Auflösung der löslichen Matrix werden gebundene stimulierte Leukozyten wieder freigesetzt und gelangen in den Körper. Gleichzeitig gelangen durch das Auflösen der Matrix tiefer liegende Schichten der Matrix mit antigenen Determinanten an die Oberfläche, und neue unbesetzte Bindungsstellen werden frei. Der Vorgang der Antigenvermittelten Leukozytenbindung und -stimulation läuft ununterbrochen weiter, bis keine lösliche Matrix mehr vorhanden ist.

Vorteilhafterweise kann durch Einsatz des erfindungsgemäßen Moduls die Akkumulation von Leukozyten und ein immunstimulierender Mechanismus in einem definierten Volumen erreicht werden. Bei einer ex vivo-Anwendung gemäß Stand der Technik werden stimulierte Leukozyten in den Körper des Patienten zurückgegeben, wobei die Verteilung der stimulierten Leukozyten nach der Rückgabe vollkommen unklar ist.

Durch den Einsatz des Leukozytenstimulations-Moduls (d.h. insbesondere durch Einbringen in den Blutstrom des Patienten), oder auch durch ex vivo-Inkubation der Matrix mit Vollblut des Patienten, binden zirkulierende Leukozyten, z.B. T-Zellen, spezifisch an die äußere Schicht der löslichen Matrix. Die erfindungsgemäße Zusammensetzung der löslichen Matrix bedingt den sukzessiven Abbau der äußeren Schichten. Dadurch werden auch gebundene und stimulierte Leukozyten vom Festkörper abgelöst und werden durch den Blutfluss oder durch mechanische Behandlung (z.B. Schütteln) dem Blut zurückgeführt. Das Ablösen der äußeren Schicht der Hülle bewirkt die Erneuerung der immunogenen Oberfläche. Leukozyten können an die neu an der Oberfläche erschienenen immunstimulatorischen Komplexe, Antigene, Zellbestandteile, MHC-Moleküle etc. binden. Der Vorgang wiederholt sich solange, bis die Hülle vollständig aufgelöst ist. Die abgelösten Bestandteile sind in Blut untoxisch und ungefährlich, da es sich ausschließlich um biokompatibles Material und/oder autologe Bestandteile des Patientenhandelt. Die möglicherweise weiterbestehende Immunogenität der zirkulierenden immunstimulatorischen Komplexe im Blut ist zusätzlich von Vorteil, da es bis zu deren biologischem Abbau zur weiteren antigenspezifischen Immunstimulation im Patienten kommt.

Das Modul weist bevorzugt ein Kunststoffgehäuse mit bevorzugt etwa 5 bis 500 ml Volumen auf. Ein- und Ausströmungsstutzen werden im Durchmesser an die Schlauchverbindungen der Katheter-Anschlüsse angepasst. Der Katheter kann doppellumig (z.B. ein Sheldon-Katheter) sein, so dass es ermöglicht wird, die Fließgeschwindigkeit des Blutes pro Lumen um bis zu 50% zu vermindern. Das Gehäuse beinhaltet die erfindungsgemäße Leukozytenstimulations-Matrix.

Bei Bedarf kann das Leukozytenstimulations-Modul als funktionelle Einheit nach Befüllung mit Blut des Patienten von den Katheterverbindungen abgekoppelt werden, um z.B. weitere Modifikation ex vivo vorzunehmen (z.B. Zugabe von kostimulatorischen Faktoren, Zytokinen, Hormonen, inhibierenden Faktoren usw.), ohne dabei das Blut aus dem Modul zu entnehmen. Das mit Blut gefüllte Gehäuse kann für einige Stunden bei stetiger Bewegung und Temperaturen über 30°C inkubiert werden, um es dann nach dem Patienten über den bereits liegenden Katheter direkt aus dem Leukozytenstimulations-Modul zurück zuzuführen. Dies kann auch mehrfach wiederholt werden.

### Vorteile der Erfindung

Es existieren derzeit keine Verfahren zur zeitlich kontrollierten antigenspezifischen Leukozytenstimulation im Blutfluss bzw. im Vollblut oder allgemein in Leukozyten enthaltenden Flüssigkeiten. Durch die vorliegende Erfindung konnte das Problem gelöst werden, dass bei der Stimulation von Leukozyten über immobilisierte Antigene gemäß Stand der Technik eine derartig stabile Bindung an den Festkörper stattfindet, dass eine Ablösung der Leukozyten nicht mehr gewährleistet ist. Im Blutkreislauf kann dies zur Anhäufung der Leukozyten innerhalb des eingesetzte Moduls gemäß Stand der Technik führen. Die Folge ist eine Erhöhung des Widerstands und spezifischer Stress für die Leukozyten, unerwünschte Nebenreaktionen, erhöhte Gerinnungseffekte. Die kontinuierliche Rückführung der stimulierten Leukozyten in den Blutkreislauf gemäß der vorliegenden Erfindung ermöglicht die Lösung der obengenannten Schwierigkeiten.

Das Leukozytenstimulations-Modul bzw. die Leukozytenstimulations-Matrix gemäß er vorliegenden Erfindung können bei der Therapie gegen virale chronische Infektionserkrankungen eingesetzt werden. Die Chronizität basiert auf der Persistenz des Antigens im Gewebe und deren unzureichenden Immunantwort dagegen. Virale Erkrankungen werden zumeist mit Chemotherapeutika therapiert. Dies hat häufig eine Reminiszenzbildung der Nieren und starke Nebenwirkungen zur Folge. Durch die vorliegende Erfindung können de novo eine Induktion bzw. Verstärkung einer hoch-spezifischen Immunantwort gegen ein Pathogen gewährleistet werden.

Durch die Bindung an den oder die Bestandteile zur Generierung einer Leukozytenstimulation und/oder Induktion einer immunologischen Toleranz können bereits geprägte Immunzellen spezifisch in ihrer Immunantwort verstärkt werden. Diese in niedriger Frequenz im Blut enthaltenen Zellen müssen dabei nicht aufwendig isoliert und ex vivo expandiert werden, da die Zellen über den Blutstrom automatisch in das Leukozytenstimulations-Modul gelangen und dort stimuliert werden. Nach ihrer Aktivierung gelangen die Leukozyten durch die Auflösung der löslichen Matrix als Effektorzellen direkt in den Blutkreislauf und in die betroffenen Gewebe. Durch die physiologische Umgebung im Blutstrom ist gewährleistet, dass alle notwendigen Faktoren für eine Ausreifung zu hochaktiven Effektorzellen vorhanden sind.

Überdies können gemäß der vorliegenden Erfindung auch Neuimmunisierungen vorgenommen werden und Toleranzinduktionen hervorgerufen werden.

Die Erfindung betrifft weiterhin die Verwendung einer erfindungsgemäßen Leukozytenstimulations-Matrix oder eines erfindungsgemäßen Leukozytenstimulations-Moduls zur Leukozytenstimulation und/oder Induktion einer immunologischen Toleranz, sowie deren Verwendung in Nachweisverfahren der Verteilung aktivierter T-Zell-Subtypen oder für Impfungen.

Die vorliegende Erfindung wird im folgenden anhand von Ausführungsbeispielen beschrieben, die den Umfang der vorliegenden Erfindung jedoch nicht beschränkten sollen.

### 1. Ausführungsform mit in der löslichen Matrix eingebetteten Antigenen

Der Cytomegalievirusstamm Hi91 wurde in humanen kultivierten Vorhautfibroblasten gezüchtet. Nach Auftreten des zytopathischen Effekts wurden die Zellkulturüberstände gesammelt, die Viren mittels Ultrazentrifugation angereichert, mit Phosphat-gepufferter Salzlösung (0,1 M PBS mit Ca²⁺, Mg²⁺, pH 7,4) gewaschen und mittels UV-Bestrahlung inaktiviert. Die Viren wurden mit 100 µl Natriumbicarbonat-Puffer (50 mM; pH 8,0-9,6), der 5 Gew.-% 15-20 kD PEG (Produkt Nr. P2263, Sigma-Aldrich, 2,2'-([Methylethyliden]-bis[4,1-penylenoxymethylen])-bis-oxiran-Polymer mit α-Hydroxypoly(oxy-1,2-ethandiyl) enthielt, kurz (3-5 Minuten) bei Raumtemperatur inkubiert (PEGiliert). 10 mg offenporiger Polyurethan (PU)-Schaum (PU Medical Grade, Firma KCI) wurden mit den in der Reaktionsmischung enthaltenen PEGilierten Viren bei Raumtemperatur inkubiert und anschließend getrocknet.

Dabei wurden 20 µl der Antigenpräparation (CMV Hi91) beigemischt. Für die Stimulationsversuche wurde Antigen von 1x10⁶-1x10⁷ Viren verwendet. Anschließend wurde Vollblut (500 µl) eines CMV positiven Spenders mit bekannter nachweisbarer dauerhafter CMV-spezifischer zellulärer Immunaktivität den vorbehandelten Materialproben zugegeben, und es wurde für eine Stunde in Bewegung bei 37° C inkubiert.

Nach dem Waschen der Träger mit dem obigen Natriumbicarbonat-puffer wurde das Blut für die durchflusszytometrische Analyse (FACS) zum Nachweis von *de novo* generierten proinflämmatorischen Zytokinen in T-Zellen (CD4 und CD8) vorbereitet. Für den Nachweis der T-Zell Aktivierung (CD69/IFN-gamma) wurde ein käuflicher Standard-Testkit (Becton Dickinson) verwendet.

### 2. Ausführungsformen mit kovalent gebundenen Antigenen

Die Versuche wurden wie oben unter 1. beschrieben durchgeführt. Allerdings wurde das Antigen nicht zusammen mit PEG zugegeben, sondern der PU-Schaum wurde vor Kontaktieren mit PEG und der Cellulose für 1-2 Stunden mir dem Bindungskoppler 3-(Triethoxysilyl)propylbernsteinsäureanhydrid (5 % Geniosil® GF20 in Methanol) vorbehandelt. Anschließend wurden wie oben vorbereitete Antigene aus der Hülle des Cytomegalievirus auf die Trägermaterialien durch Inkubation kovalent gebunden.

### Ergebnisse:

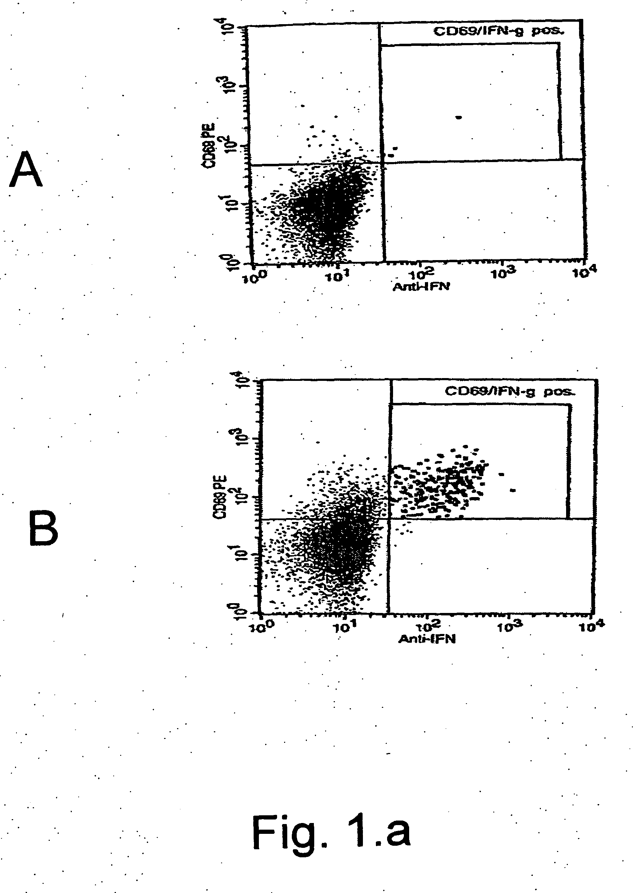

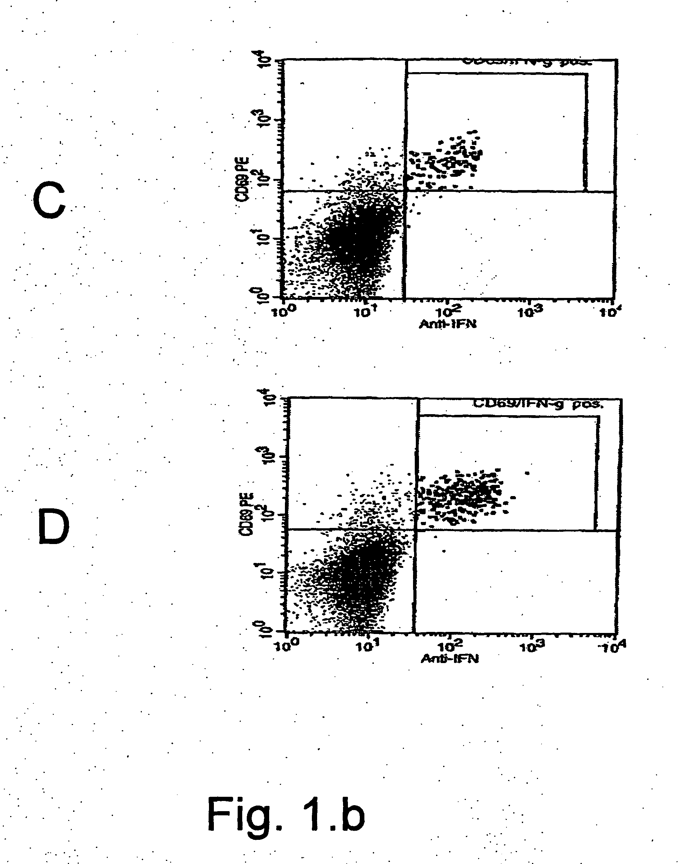

Sowohl kovalent gebundene als auch in der löslichen Matrix eingebettete Antigene konnten im Vollblut eine T-Zell vermittelte Immunantwort auslösen. Der Träger mit den kovalent gebundenen Antigenen zeigte nach mehrfach hintereinander wiederholten Ansätzen eine zunehmend abgeschwächte Immunreaktion (CD69/IFN-γ). Dagegen war die generierte Immunreaktion mittels löslicher Matrix über die Zeit konstant. Abbildung 1 zeigt beispielhaft eine Darstellung der durchflusszytometrischen Versuchsergebnisse zur Stimulation der CMV-spezifischen Immunantwort (Aktivierungsmarker CD69/Interferon-gamma Produktion) mittels kovalent gebundener CMV-Antigene (Polyurethan KCI Medical Grade mit Silan (5% Geniosil® in Methanol) vorbehandelt) sowie nicht kovalent eingebetteter CMV-Antigene. Die Ausschüttung der Zytokine wurde durch Vorbehandlung der Zellen mit Brefeldin A gehemmt. Die Quadrantenanalyse zeigt nach Abzug der Kontrollwerte (0,02 % unstimulierte Zellen) eine spezifische Aktivierung bei 2,49 % der Lymphozyten (CD4⁺) .

Auf einem Träger kovalent oder in einer löslichen Matrix eingebettete Antigene können somit eine spezifische T-Zell vermittelte Immunantwort im Vollblut auslösen. Die lösliche Matrix führt zu einer kontinuierlichen Erneuerung der Antigen stimulierenden Komponente, die im Kontakt mit dem Blut ist. Blutbestandteile können demnach die funktionelle Kapazität nicht inhibieren.

Fig. 1.a und b: Durchflusszytometrische Ergebnisse, als Quadranten-analyse dargestellt. Die Punktewolke im Kasten rechts oben repräsentiert jeweils die durch CMV-HI91-Antigen aktivierten (CD69/IFN-γ)CD4+ Zellen im Vollblut eines gesunden CMV-positiven Spenders.
A) unstimulierte Kontrolle: 0.02 %
B) CMV-Hi91-Antigen auf mit Silan behandeltem Polyurethan: 2.49 %
C) CMV-Hi91-Antigen auf mit Silan behandeltem Polyurethan (Wiederholung): 0.75 %
D) CMV-Hi91-Antigen auf löslicher Matrix (nach Wiederholung): 2.02 %

Obige Versuche wurden auch mit anderen Trägern wiederholt, d.h. mit Sepharose, Glas und Polystyrol, wobei vergleichbare Ergebnisse erzielt wurden.

## Patentansprüche

1. Leukozytenstimulations-Matrix zur Leukozytenstimulation und/oder Induktion einer immunologischen Toleranz, mit folgenden Bestandteilen:
a) ein oder mehreren Trägern,
b) einer löslichen Matrix zur Einbettung eines oder mehrerer Bestandteile zur Generierung einer Leukozytenstimulation und/oder Induktion einer immunologischen Toleranz,
c) ein oder mehreren in die lösliche Matrix eingebetteten Bestandteilen zur Generierung einer Leukozytenstimulation und/oder Induktion einer immunologischen Toleranz.

2. Leukozytenstimulations-Matrix nach Anspruch 1, wobei zusätzlich ein oder mehrere Bindungskoppler vorgesehen sind, um eine Bindung zwischen Träger und den ein oder mehreren Bestandteilen zur Generierung einer Leukozytenstimulation und/oder Induktion einer immunologischen Toleranz zu vermitteln.

3. Leukozytenstimulations-Matrix nach Anspruch 2, wobei die Bindung eine kovalente Bindung ist.

4. Leukozytenstimulations-Matrix nach einem der vorhergehenden Ansprüche, wobei der Bestandteil zur Generierung einer Leukozytenstimulation und/oder Induktion einer immunologischen Toleranz aus der Gruppe ausgewählt ist, die aus Antigenen, MHC-Molekülen, kostimulatorischen Faktoren, Zellbestandteilen, Zellhüllen, Bakterien, Viren und Kombinationen hiervon besteht.

5. Leukozytenstimulations-Matrix nach Anspruch einem der vorhergehenden Ansprüche, wobei der Bestandteil zur Generierung einer Leukozytenstimulation und/oder Induktion einer immunologischen Toleranz ein synthetisches Antigen ist, oder aus Viren, Bakterien, Pilzen, Tumoren, Allergenen oder aus körpereigenem Gewebe gewonnen wird, und/oder das MHC-Molekül und der kostimulatische Faktor aus körpereigenem Gewebe, aus Zellkulturen und/oder synthetisch gewonnen werden.

6. Leukozytenstimulations-Matrix nach Anspruch 4 oder 5, wobei der Bestandteil zur Generierung einer Leukozytenstimulation und/oder Induktion einer immunologischen Toleranz ein Virus der Familie der Herpesviren oder ein Fragment hiervon ist.

7. Leukozytenstimulations-Matrix nach einem der vorhergehenden Ansprüche, wobei der Träger aus Gruppe ausgewählt ist, die aus Polyurethanen, Polycarbonaten, Polystyrol, in der Chirurgie verwendeten, sich auflösenden Materialien, Glas, Naturstoffen wie Därmen oder biologischen Materialien wie Schwämmen, oder Kombinationen hiervon besteht.

8. Leukozytenstimulations-Matrix nach einem der vorhergehenden Ansprüche 2 bis 7, wobei der Bindungskoppler aus der Gruppe ausgewählt ist, die aus Cyanogenbromid, Cyanoborhydrid, Agarose, Agarosederivaten, Silan, Silanderivaten oder Kombinationen hiervon besteht.

9. Leukozytenstimulations-Matrix nach Anspruch 8, wobei das Silanderivat ein Alkoxysilan ist, bevorzugt ein Anhydridoalkoxysilan oder ein anderes Alkoxysilan, welches mindestens eine Carboxylgruppe aufweist.

10. Leukozytenstimulations-Matrix nach einem der vorhergehenden Ansprüche, wobei die lösliche Matrix aus langkettigen Zuckerverbindungen wie Stärke, Cellulose, Glykogen einerseits, und/oder Polyethylenglykol andererseits aufgebaut ist.

11. Leukozytenstimulations-Matrix nach Anspruch 10, wobei die lösliche Matrix aus 50-90 Ges.-% der langkettigen Zuckerverbindung und 10-50 Gew.-% Polyethylenglykol, bezogen auf die Summe aus langkettiger Zuckerverbindung und Polyethylenglykol, aufgebaut ist.

12. Leukozytenstimulations-Modul, umfassend ein Gehäuse mit mindestens einer Öffnung sowie eine darin befindliche Leukozytenstimulations-Matrix nach einem der vorhergehenden Ansprüche.

13. Leukozytenstimulations-Modul nach Anspruch 12, umfassend mindestens eine Einlassöffnung und mindestens eine Auslassöffnung.

14. Verfahren zur Leukozytenstimulation und/oder Induktion einer immunologischen Toleranz, dadurch gekennzeichent, dass eine Leukozyten enthaltende Flüssigkeit ex vivo mit einer Leukozytenstimulations-Matrix nach einem der Ansprüche 1 bis 11 in Kontakt gebracht wird.

15. Verfahren nach Anspruch 14, wobei das Kontaktieren in einem Leukozytenstimulations-Modul nach Anspruch 12 oder 13 erfolgt.

16. Verwendung einer Leukozytenstimulations-Matrix nach einem der Ansprüche 1 bis 11 oder eines Leukozytenstimulations-Moduls nach Anspruch 12 oder 13 ex vivo zur Leukozytenstimulation und/oder Induktion einer immunologischen Toleranz.

17. Verwendung einer Leukozytenstimulations-Matrix nach einem der Ansprüche 1 bis 11 oder eines Leukozytenstimulations-Moduls nach Anspruch 12 oder 13 ex vivo in Nachweisverfahren der Verteilung aktivierter T-Zell-Subtypen oder für Impfungen.

## Claims

1. Leukocyte stimulation matrix for the stimulation of leukocytes and/or the induction of an immunological tolerance having the following components:
a) one or more carrier(s),
b) a soluble matrix for embedding one or more component(s) for generating a leukocyte stimulation and/or the induction of an immunological tolerance,
c) one or more component(s) embedded into the soluble matrix for generating a leukocyte stimulation and/or the induction of an immunological tolerance.

2. Leukocyte stimulation matrix according to claim 1 which additionally contains one or more coupling component(s) for mediating the binding between the carrier and the one or more component(s) for generating a leukocyte stimulation and/or the induction of an immunological tolerance.

3. Leukocyte stimulation matrix according to claim 2, wherein the binding is a covalent binding.

4. Leukocyte stimulation matrix according to one of the preceding claims, wherein the component for generating a leukocyte stimulation and/or the induction of an immunological tolerance is selected from the group consisting of antigens, MHC molecules, co-stimulatory factors, cell components, cell coatings, bacteria, viruses and combinations thereof.

5. Leukocyte stimulation matrix according to one of the preceding claims, wherein the component for generating a leukocyte stimulation and/or the induction of an immunological tolerance is a synthetic antigen or is obtained from viruses, bacteria, fungi, tumours, allergens or endogenous tissue, and/or the MHC molecule and the co-stimulatory factor are obtained from endogenous tissue, cell cultures and/or synthetically.

6. Leukocyte stimulation matrix according to claim 4 or 5, wherein the component for generating a leukocyte stimulation and/or the induction of an immunological tolerance is a virus of the family of herpes viruses or a fragment thereof.

7. Leukocyte stimulation matrix according to one of the preceding claims, wherein the carrier is selected from the group consisting of polyurethanes, polycarbonates, polystyrene, dissolvable materials used in surgery, glass, natural materials such as gut skins or biological materials such as sponges or combinations thereof.

8. Leukocyte stimulation matrix according to one of claims 2 to 7, wherein the coupling component is selected from the group consisting of cyanogen bromide, cyanoboro hydride, agarose, agarose derivatives, silane, silane derivatives or combinations thereof.

9. Leukocyte stimulation matrix according to claim 8 wherein the silane derivative is an alkoxy silane, preferably an anhydroalkoxy silane or another alkoxy silane having at least one carboxyl group.

10. Leukocyte stimulation matrix according to any one of the preceding claims, wherein the soluble matrix is made of long chain sugar compounds such as starch, cellulose, glycogen on the one hand and/or polyethylene glycol on the other hand.

11. Leukocyte stimulation matrix according to claim 10, wherein the soluble matrix is made of 50-90 wt.% of a long chain sugar compound and 10-50 wt.% of polyethylene glycol, based on the total of long chain sugar compound and polyethylene glycol.

12. Leukocyte stimulation module comprising a housing with at least one opening and a leukocyte stimulation matrix according to any one of the preceding claims contained therein.

13. Leukocyte stimulation module according to claim 12 comprising at least one inlet opening and at least one outlet opening.

14. A process for the stimulation of leukocytes and/or the induction of an immunological tolerance **characterised in that** a leukocyte containing liquid is contacted *ex vivo* with a leukocyte stimulation matrix according to any one of claims 1 to 11.

15. A process according to claim 14, wherein the contacting is carried out in a leukocyte stimulation module according to claim 12 or 13.

16. The *ex vivo* use of a leukocyte stimulation matrix according to any one of claims 1 to 11 or a leukocyte stimulation module according to claim 12 or 13 for the stimulation of leukocytes and/or the induction of an immunological tolerance.

17. The *ex vivo* use of a leukocyte stimulation matrix according to any one of claims 1 to 11 or a leukocyte stimulation module according to 12 or 13 in a method for detecting distribution of activated T-cell subtypes or for vaccinations.

## Revendications

1. Matrice de stimulation de leucocytes destinée à la stimulation de leucocytes et/ou l'induction d'une tolérance immunologique, comportant les composants suivants :
a) un ou plusieurs supports,
b) une matrice soluble pour inclusion d'un ou plusieurs composants destinés à produire une stimulation leucocytaire et/ou à induire une tolérance immunologique,
c) un ou plusieurs composants inclus dans la matrice solide destinés à produire une stimulation leucocytaire et/ou induire une tolérance immunologique.

2. Matrice de stimulation de leucocytes selon la revendication 1, un ou plusieurs coupleurs de liaison étant additionnellement prévus pour réaliser une liaison entre le support et le ou les composants destinés à produire une stimulation leucocytaire et/ou induire une tolérance immunologique.

3. Matrice de stimulation de leucocytes selon la revendication 2, la liaison étant une liaison covalente.

4. Matrice de stimulation de leucocytes selon l'une des revendications précédentes, le composant destiné à produire une stimulation des leucocytes et/ou induire une tolérance immunologique étant choisi dans le groupe constitué d'antigènes, de molécules de CMH, de facteurs co-stimulants, de composants cellulaires, d'enveloppes cellulaires, de bactéries, de virus et leurs combinaisons.

5. Matrice de stimulation de leucocytes selon l'une quelconque des revendications précédentes, le composant destiné à produire une stimulation des leucocytes et/ou induire une tolérance immunologique étant un antigène de synthèse ou étant obtenu à partir de virus, de bactéries, de champignons, de tumeurs, d'allergènes ou de tissu endogène, et/ou la molécule de CHM et le facteur co-stimulant étant obtenus à partir de tissu endogène, de cultures cellulaires et/ou par synthèse.

6. Matrice de stimulation de leucocytes selon la revendication 4 ou 5, le composant destiné à produire une stimulation leucocytaire et/ou induire une tolérance immunologique étant un virus de la famille des herpèsvirus ou un fragment de celui-ci.

7. Matrice de stimulation des leucocytes selon l'une des revendications précédentes, le support étant choisi dans le groupe constitué de polyuréthanes, polycarbonates, polystyrène, matériaux solubles utilisés en chirurgie, verre, substances naturelles telles que des intestins ou matériaux biologiques tels que des éponges, ou leurs combinaisons.

8. Matrice de stimulation de leucocytes selon l'une des revendications 2 à 7 précédentes, le coupleur de liaison étant choisi dans le groupe constitué du bromure de cyanogène, du cyanohydroborure, de l'agarose, de dérivés de l'agarose, du silane, de dérivés du silane ou leurs combinaisons.

9. Matrice de stimulation de leucocytes selon la revendication 8, le dérivé de silane étant un alcoxysilane, de préférence un anhydridoalcooxylsilane ou un autre alcoxysilane présentant au moins un groupe carboxyle.

10. Matrice de stimulation de leucocytes selon l'une des revendications précédentes, la matrice soluble étant constituée de composés glucidiques à chaîne longue tels que l'amidon, la cellulose, le glycogène d'une part et/ou de polyéthylèneglycol d'autre part.

11. Matrice de stimulation de leucocytes selon la revendication 10, la matrice solide étant constituée de 50 à 90 % en poids du composé glucidique à chaîne longue et de 10 à 50 % en poids de polyéthylèneglycol, par rapport au total du composé glucidique à chaîne longue et du polyéthylèneglycol.

12. Module de stimulation de leucocytes comprenant un boîtier ayant au moins une ouverture ainsi qu'une matrice de stimulation de leucocytes selon l'une des revendications précédentes qui est contenue.

13. Module de stimulation de leucocytes selon la revendication 12, comprenant au moins une ouverture d'entrée et au moins une ouverture de sortie.

14. Procédé de stimulation de leucocytes et/ou d'induction d'une tolérance immunologique, **caractérisé en ce qu'**un liquide contenant des leucocytes est amené ex vivo en contact avec une matrice de stimulation des leucocytes selon l'une des revendications 1 à 11.

15. Procédé selon la revendication 14, la mise en contact ayant lieu dans un module de stimulation des leucocytes selon la revendication 12 ou 13.

16. Utilisation d'une matrice de stimulation des leucocytes selon l'une des revendications 1 à 11, ou d'un module de stimulation des leucocytes selon la revendication 12 ou 13 ex vivo pour la stimulation de leucocytes et/ou l'induction d'une tolérance immunologique.

17. Utilisation d'une matrice de stimulation des leucocytes selon l'une des revendications 1 à 11, ou d'un module de stimulation des leucocytes selon la revendication 12 ou 13 ex vivo dans un procédé de mise en évidence de la répartition de sous-types de lymphocytes T activés ou pour des vaccinations.
